# EUROPÄISCHE PATENTSCHRIFT

(11) **EP 1 214 954 B1**
(45) Veröffentlichungstag und Bekanntmachung des Hinweises auf die Patenterteilung: **02.11.2006**
(21) Anmeldenummer: 01128566.5
(22) Anmeldetag: 30.11.2001
(51) Int. Cl.: A61M 1/10, A61M 5/168, A61M 5/142

(54) **Pumpeneinrichtung für eine medizinische Flüssigkeit sowie medizinische Behandlungseinrichtung zum Zuführen einer Flüssigkeit zum menschlichen oder tierischen Körper**
Medical fluid pump and medical treatment device for fluid delivery to the human or animal body
Pompe à liquides à usage médical et dispositif de traitment pour administrer un fluide dans le corps d'un humain ou d'un animal

(30) Priorität: 12.12.2000 DE 20021033 U; 17.05.2001 DE 20108350 U; 16.08.2001 DE 10140214
(43) Veröffentlichungstag der Anmeldung: 19.06.2002
(73) Patentinhaber: Bösherz, Jakob, 94116 Hutthurm (DE); Götz, Alois H., Dr., 91257 Pegnitz (DE)
(72) Erfinder: Bösherz, Jakob, 94116 Hutthurm (DE); Götz, Alois H., Dr., 91257 Pegnitz (DE)
(74) Vertreter: Blaumeier, Jörg

(56) Entgegenhaltungen:
- EP-A- 0 650 738
- DE-C- 740 826
- US-A- 4 207 871
- US-A- 4 250 872
- US-A- 4 459 977
- US-A- 4 493 709
- US-A- 4 552 552
- US-A- 4 643 713
- US-A- 5 061 236

## Beschreibung

Die Erfindung betrifft eine Pumpeneinrichtung zum Pumpen einer Flüssigkeit, insbesondere einer einem menschlichen oder tierischen Körper zuzuführenden Flüssigkeit, insbesondere von Blut.

Eine Pumpeneinrichtung gemäß dem Oberbegriff von Anspruch 1 ist aus der EP-A-0 650 738 bekannt.

Pumpeneinrichtungen zum Pumpen einer Flüssigkeit werden manigfaltig verwendet. Bekannt sind dabei u.a. sogenannte Rollenpumpen, die im medizinischen Bereich insbesondere zum Pumpen von Blut, z.B. in einem Dialysegerät, verwendet werden. Solche Pumpen umfassen einen im Wesentlichen U-förmig geführten, an einem Gehäuseabschnitt gelagerten Schlauch, in dem die zu fördernde Flüssigkeit, also beispielsweise Blut geführt ist, sowie zwei oder mehrere Roller, die sich in dem U-förmigen Schlauchabschnitt drehen, an dem elastischen Schlauch von außen angreifen und ihn mit einstellbarer Geschwindigkeit kontinuierlich in einer Richtung komprimieren. Durch dieses Komprimieren wird die Flüssigkeitsmenge gefördert. Nachteilig bei dieser Einrichtung ist aber beim Pumpen von Blut, dass durch das Zusammendrücken des relativ dünnen elastischen Schlauchs die Blutzellen zwischen den dicht zusammenkommenden Schlauchwänden gequetscht und erheblich geschädigt werden können. Dies kann im schlimmsten Fall zur Hämolyse und Anämie führen. Solche Probleme sind jedoch nicht nur beim Einsatz auf medizinischem Sektor gegeben, sondern überall dort, wo sensible Stoffe mittels derartiger Pumpen gefördert werden. Exemplarisch ist hierbei z.B. der Arzneimittelsektor zu nennen, wo ebenfalls häufig sehr sensible Stoffe gefördert werden.

Der Erfindung liegt dabei das Problem zugrunde, eine Pumpeneinrichtung anzugeben, die hier Abhilfe schafft.

Zur Lösung dieses Problems ist eine Pumpeneinrichtung gemäß den Merkmalen von Anspruch 1 vorgesehen.

Die Erfindung sieht mit besonderem Vorteil einen ballonartigen, elastisch komprimierbaren Abschnitt innerhalb des die Flüssigkeit fördernden Schlauchs vor, der im Vergleich zum Durchmesser des Schlauches deutlich größer ist. Dieser mit der Flüssigkeit, also beispielsweise Blut gefüllte ballonartige Abschnitt wird über ein Komprimiermittel zusammengedrückt, wodurch sich sein Volumen verringert und die darin befindliche Flüssigkeit aus ihm herausgedrückt wird. Da das Volumen des ballonartigen Abschnitts in unkomprimiertem Zustand wesentlich größer ist als das Volumen eines entsprechend langen, geradlinigen Schlauchabschnitts, ist es im Vergleich zum Stand der Technik problemlos möglich; die gleiche oder eine größere Menge zu fördern, ohne dass der ballonartige Abschnitt sehr stark komprimiert werden muss. Die Fördermenge lässt sich durch entsprechend starkes Komprimieren selbstverständlich hinreichend erhöhen. Eine sensible Flüssigkeit kann nun unter Verwendung der erfindungsgemäßen Pumpeneinrichtung gefahrlos gefördert werden, da zum Fördern einer hinreichenden Menge der ballonartige Abschnitt nicht allzu sehr verformt werden muss, d.h. die Abschnittswände müssen nicht allzu nah zueinander geführt werden während des Komprimierens. Eine Beeinträchtigung der sensiblen Blutkörperchen oder anderer Flüssigkeitsbestandteile ist unter Verwendung der erfindungsgemäßen Pumpeneinrichtung mithin ausgeschlossen.

Im Hinblick auf eine hohe Fördermenge ist es zweckmäßig, wenn erfindungsgemäß zwei schlauchartige Leitungsabschnitte mit je einem ballonartigen, elastisch komprimierbaren Abschnitt vorgesehen sind, die von einer gemeinsamen Zufuhrleitung abgehen und die in eine gemeinsame Ableitung münden. Dabei kann vorgesehen sein, dass den beiden elastisch komprimierbaren Abschnitten je ein separates Komprimiermittel zugeordnet ist. Alternativ dazu können auch beide über ein gemeinsames Komprimiermittel betätigt werden. Die beiden Abschnitte können dabei nach einer ersten Erfindungsausgestaltung über das oder die Komprimiermittel gleichzeitig betätigt werden, d.h. die Abschnitte werden gleichzeitig zusammengedrückt. Um eine im Wesentlichen kontinuierliche Förderung zu erzielen ist es demgegenüber möglich, die Abschnitte auch wechselweise oder alternierend zu komprimieren.

Erfindungsgemäß ist vorgesehen, daß dann, wenn ein Abschnitt über das Komprimiermittel komprimiert wird, die einander gegenüberliegenden Abschnittswände auch bei starker Kompression nicht aneinandergedrückt werden. Um dem Rechnung zu tragen weist das Komprimiermittel ein am ballonartigen Abschnitt außenseitig angreifendes bewegbares Druckstück auf, dessen Außenform im am Abschnitt angreifenden Bereich im Wesentlichen der expandierten Form des gegenüberliegenden Wandabschnitts des ballonartigen Abschnitts entspricht. Ist also der ballonartige Abschnitt kugelartig ausgebildet, so ist die Außenform des Druckstücks halbkugelartig.

Bei zwei schlauchartigen Leitungsabschnitten mit je einem ballonartigen elastisch komprimierbaren Abschnitt sind diese zweckmäßigerweise einander gegenüberliegend angeordnet, wobei zwischen ihnen ein oder zwei bewegbare Druckstücke angeordnet sind. Ist ein bewegbares Druckstück vorgesehen, so ist ein wechselweises Komprimieren der ballonartigen Abschnitte möglich, sind zwei vorgesehen, so ist sowohl ein gleichzeitiges wie auch ein wechselseitiges Komprimieren je nach gewählter Ansteuerungsart denkbar.

Zum Steuern des Betriebs des oder der Komprimiermittel ist zweckmäßigerweise eine entsprechende Steuerungseinrichtung vorgesehen, über die die Frequenz und/oder Amplitude (=Komprimierung) der Komprimierbewegungen des oder der Komprimiermittel variierbar ist. An der Steuerungseinrichtung können ferner Mittel zum Wählen und Einstellen der Betriebsparameter des oder der Komprimiermittel und/oder von für den Betrieb des oder der Komprimiermittel relevanten Parametern, insbesondere des Körpergewichts des Menschen oder des Tieres vorgesehen sein. Die Eingabe eines oder mehrerer derartiger Parameter ist insbesondere hinsichtlich einer Bestimmung der zu fördernden Menge, z.B. bei der Dialyse der zu fördernden Blutmenge zweckmäßig, was wiederum Einfluss auf die Frequenz sowie gegebenenfalls auch auf die Amplitude der Komprimierbewegung, also auf den Weg, um den ein ballonartiger Abschnitt zusammengedrückt wird, haben kann.

Ein Leitungsabschnitt samt einem ballonartigen Abschnitt ist zweckmäßigerweise aus PVC gebildet, wobei dieses Kunststoffmaterial eine hinreichende Elastizität bietet.

Um den Fluss der zu pumpenden Flüssigkeit hinsichtlich der Förderrichtung steuern zu können ist es zweckmäßig, wenn vor und/oder hinter dem ballonartigen Abschnitt Mittel zum reversiblen, zumindest teilweisen Schließen und Öffnen des Leitungsabschnitts vorgesehen sind. Diese Mittel, die synchron mit den Komprimiermitteln arbeiten, ermöglichen es zu entsprechenden Zeitpunkten den Leitungsabschnitt je nach Bedarf vor oder hinter dem Ballonabschnitt zu sperren. Soll beispielsweise der ballonartige Abschnitt komprimiert werden muss verhindert werden, dass die Flüssigkeit entgegen der eigentlichen Förderrichtung zurückgedrängt werden kann. In diesem Fall wird hinter dem ballonartigen Abschnitt der Leitungsabschnitt weitgehend verschlossen. Ist der ballonartige Abschnitt komprimiert, so muss verhindert werden, dass beim Lösen des Komprimierdrucks und beim folglich eintretenden selbsttätigen Entspannen des Abschnitts die bereits in die gewünschte Förderrichtung geförderte Flüssigkeit während dieser Entspannungsbewegung zurückgesaugt wird. In diesem Fall wird der Leitungsabschnitt vor dem ballonartigen Abschnitt zumindest teilweise geschlossen.

Bei zwei Leitungsabschnitten mit je einem ballonartigen Abschnitt sind zweckmäßigerweise vor und hinter dem jeweiligen ballonartigen Abschnitt derartige Mittel vorgesehen. Im Falle einer wechselseitigen Komprimierung der beiden ballonartigen Abschnitte ist die Steuerung der Komprimiermittel sowie der Verschlussmittel über die Steuerungseinrichtung derart, dass das dem zu komprimierenden Abschnitt vorgeschaltete Mittel und das dem nicht zu komprimierenden Abschnitt nachgeschaltete Mittel zumindest teilweise geschlossen und die jeweils anderen Mittel geöffnet werden. Hierdurch wird vorteilhaft vermieden, dass die über den einen Leitungsabschnitt, der auch als Leitungsast bezeichnet werden kann, durch das Komprimieren gepumpte Flüssigkeit in den anderen Leitungsabschnitt umgepumpt wird, der über das dem nicht zu komprimierenden Abschnitt nachgeschaltete Mittel geschlossen ist. Dabei sind die Begriffe vor- und nachgeschaltet jeweils bezogen auf die eigentliche Förderrichtung zu verstehen.

Im Falle einer gleichzeitigen Komprimierung der beiden Abschnitte erfolgt der Betrieb über die Steuerungseinrichtung derart, dass die den Abschnitten vorgeschalteten Mitteln beim Komprimieren geschlossen und die nachgeschalteten Mittel geöffnet werden. Werden die beiden Abschnitte entlastet, so werden die nachgeschalteten Mittel geschlossen, um ein Rücksaugen zu verhindern, und die vorgeschalteten Mittel geöffnet.

Die Verschließmittel umfassen zweckmäßigerweise ein von außen auf den Leitungsabschnitt drückendes bewegbares Drückelement, das erfindungsgemäß eine im Wesentlichen keilförmige Form im Bereich seines am Leitungsabschnitt angreifenden Endes aufweisen kann. Diese Keilform ist dahingehend von Vorteil, als beim Verschließen nur ein sehr schmaler Bereich des Leitungsabschnitts zusammengedrückt wird, so dass die Gefahr einer Beschädigung von sensiblen Flüssigkeitsbestandteilen weitestgehend vermieden wird.

Aus Reinigungs- und Wartungsgesichtspunkten ist es vorteilhaft, wenn der oder die Leitungsabschnitte sowie das oder die Komprimiermittel in einem separaten Gehäuse untergebracht sind, an dem Anschlüsse für von außerhalb des Gehäuses zugeführte Zufuhr- und Abfuhrleitungen für die zu pumpende Flüssigkeit vorgesehen sind. Die zentrale Pumpeneinrichtung ist also in einem separaten Gehäuse angeordnet und kann lösbar mit den Zufuhr- und Abfuhrleitungen gekoppelt werden, so dass sie auf einfache Weise dem eigentlichen Pumpeneinrichtungsgehäuse entnommen und gereinigt oder gewartet werden kann. Dabei sind der oder die Leitungsabschnitte ebenfalls zweckmäßigerweise lösbar in dem Gehäuse angeordnet, so dass auch sie entnommen werden können und gegebenenfalls sterilisiert oder sonst wie gereinigt und behandelt werden können.

Weiterhin kann ein Einrichtungsgehäuse vorgesehen sein, in dem das die Leitungsabschnitte und die Komprimiermittel enthaltende Gehäuse lösbar angeordnet und mit dort vorgesehenen Zufuhr- und Abfuhrleitungen verbindbar ist, und in dem die Steuerungseinrichtung angeordnet ist, die an am Gehäuse vorgesehene Steuerungsanschlüsse anschließbar ist.

Neben der Pumpeneinrichtung betrifft die Erfindung ferner eine medizinische Behandlungseinrichtung zum Zuführen einer Flüssigkeit zum menschlichen oder tierischen Körper, insbesondere eine Dialyse- oder eine Infusionseinrichtung, die sich durch eine Pumpeneinrichtung der vorbeschriebenen Art auszeichnet.

Eine solche Behandlungseinrichtung, z.B. in Form einer Dialyseeinrichtung, ermöglicht ein Fördern des Blutes ohne die Gefahr, dass die Blutkörperchen oder andere sensible Flüssigkeitsbestandteile beschädigt werden können. Ein Nachteil einer solchen Behandlungseinrichtung ist jedoch, dass mitunter durch den Pumpbetrieb ein zu hoher Unterdruck am Eingang des Fördersystems anliegen kann, so dass ein Alarm ausgelöst wird und das System angehalten wird. Um hier Abhilfe zu schaffen ist vorgesehen, dass in der Zufuhrleitung der Flüssigkeit zu der Pumpeneinrichtung eine Einrichtung zum Einstellen und Begrenzen eines durch den Betrieb der Pumpeneinrichtung am Einlass der Zufuhrleitung entstehenden Unterdrucks vorgesehen oder in diese einschaltbar ist. Hierüber wird vorteilhaft der Unterdruck begrenzt, so dass die genannten Nachteile, die durch den Betrieb der erfindungsgemäßen Pumpeneinrichtung - die natürlich ein relativ großes Volumen pro Zeiteinheit pumpen kann, da der ballonartige Abschnitt ein deutlich größeres Fördervolumen darstellt als z.B. bei bekannten Rollerpumpen - vorteilhaft vermieden werden. Hierzu ist es zweckmäßig, wenn die Unterdruckeinstell- und begrenzungseinrichtung ein mit einer biokompatiblen Flüssigkeit, z.B. einer antikoagulierenden Lösung wie Heparin gefülltes Behältnis umfasst, das über eine Zuleitung mit der Zufuhrleitung in Verbindung steht und das an einer Position unterhalb der Pumpeneinrichtung angeordnet oder anbringbar ist. Der eingestellte zulässige Unterdruck wird hierbei durch die Höhendifferenz zwischen der Pumpeneinrichtung und dem Behältnis bestimmt, aus dem zwangsläufig dann die Flüssigkeit nachgesaugt wird, wenn der Unterdruck den aus der Höhendifferenz sich ergebenden zulässigen Druck übersteigt. Dabei ist es zweckmäßig, wenn ein Sensormittel zum Erfassen des Füllstands des Behältnisses vorgesehen ist, das ein Erfassungssignal liefert, in dessen Abhängigkeit ein Alarmsignal über die Steuerungseinrichtung gebbar und/oder der Betrieb der Pumpeneinrichtung steuerbar ist. Geht der Flüssigkeitspegel im Behältnis zur Neige, so wird zweckmäßigerweise ein Signal gegeben, so dass das Behältnis ausgetauscht wird.

In weiterer vorteilhafter Erfindungsausgestaltung ist vorgesehen, dass in der Zufuhrleitung zur Pumpeneinrichtung eine Luftfalle und dieser nachgeschaltet ein Sensorelement zum Ermitteln von Luft in der Flüssigkeit angeordnet ist. Vor allem im Falle eines Dialysegeräts ist es in jedem Fall zu vermeiden, dass das dem Patienten zugeführte Blut Luft enthält, da dies verheerende Folgen wie eine Embolie oder dergleichen zur Folge haben kann. Zu diesem Zweck ist es vorteilhaft, wenn der Pumpeneinrichtung vorgeschaltet eine Luftfalle und dieser nachgeschaltet wiederum ein Sensorelement, z.B. ein fotometrischer Sensor, der extern zur Flüssigkeitsleitung angeordnet ist, vorgesehen ist, wobei über das Sensorelement etwaige in der Flüssigkeit vorhandene Luft erfasst wird. Liefert der Sensor ein Erfassungssignal, so wird selbstverständlich der Betrieb der Pumpeneinrichtung und damit der Behandlungseinrichtung gestoppt und ein Alarmsignal generiert. Das Anordnen des Sensorelements nach der Luftfalle ist dahingehend zweckmäßig, als das Sensorelement die in der Luftfalle bereits von Luft befreite Flüssigkeit nochmals überprüft. Da in der Regel in der Luftfalle sämtliche in der Flüssigkeit vorhandene Restluft abgezogen werden kann, wird auf diese Weise vermieden, dass es zu häufigen Alarmen kommt. Die der Luftfalle zugeführte Flüssigkeit wird dabei zweckmäßigerweise über einen von unten her in die Luftfalle, bei der es sich um ein größeres vertikal stehendes Behältnis handelt, in dem sich oberseitig die mitgeführte Luft sammelt, von wo sie auch abgezogen werden kann, führende Zuführleitungsabschnitt zugeführt und über einen nach unten von der Luftfalle abgehenden Abfuhrleitungsabschnitt abgeführt wird.

Weitere Vorteile, Merkmale und Einzelheiten der Erfindung ergeben sich aus den im folgenden beschriebenen Ausführungsbeispielen sowie anhand der Zeichnungen. Dabei zeigen:
- Fig. 1 u. 2: die erfindungsgemäße Pumpeinrichtung als Prinzipskizze in zwei unterschiedlichen Pumpzuständen,
- Fig. 3: eine erfindungsgemäße medizinische Behandlungseinrichtung in Form eines Dialysegeräts, und
- Fig. 4: eine erfindungsgemäße Behandlungseinrichtung in Form eines Infusionsapparats.

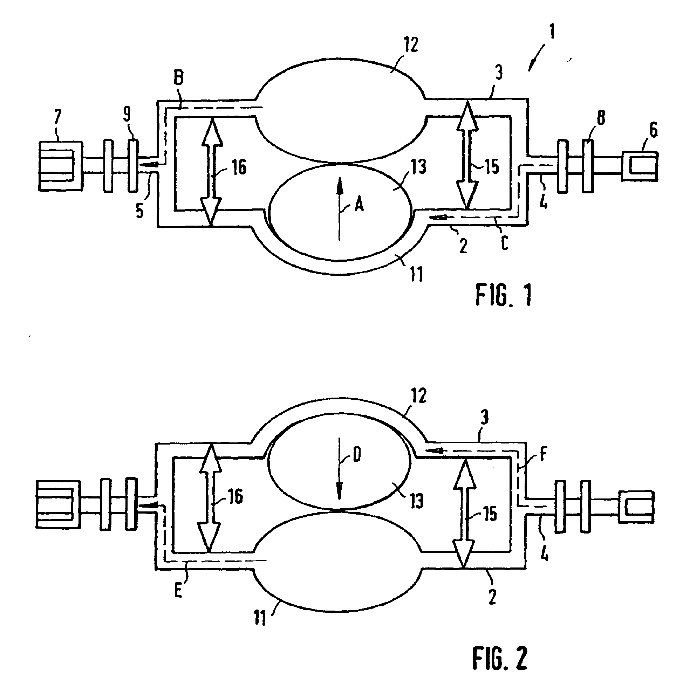

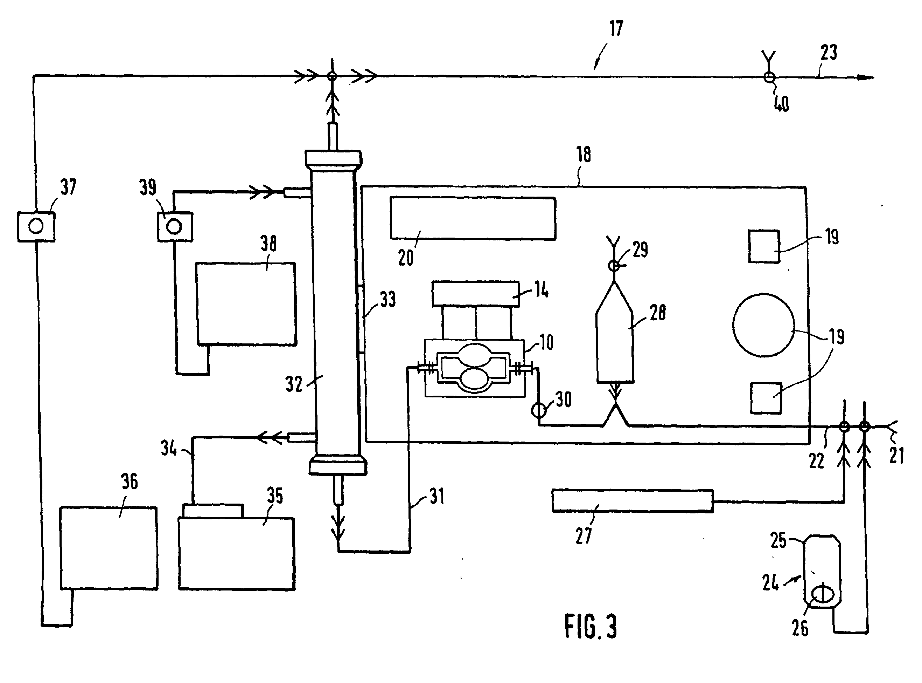

Fig. 1 zeigt eine erfindungsgemäße Pumpeneinrichtung 1 umfassend einen ersten Leitungsabschnitt 2 und einen zweiten Leitungsabschnitt 3, die in der gezeigten Prinzipskizze mit Wesentlichen parallel zueinander verlaufen und über eine gemeinsame Zufuhrleitung 4 sowie eine gemeinsame Abfuhrleitung 5 miteinander verbunden sind. An der Zufuhrleitung 4 ist ein Anschlussmittel 6 zum Anschließen an eine externe Flüssigkeitszufuhrleitung und an der Abfuhrleitung 5 ein Anschlussmittel 7 zum Anschließen an eine von der Pumpeneinrichtung abgehende externe Abfuhrleitung vorgesehen. Weiterhin sind an den Flüssigkeitszufuhr- und abfuhrleitungen 4, 5 Fixierelemente 8, 9 vorgesehen, mittels denen die Leitungskonfiguration in einem Gehäuse 10 (siehe Fig. 3) lösbar befestigt werden kann.

Wie die Figuren 1 und 2 zeigen besitzt jeder Leitungsabschnitt 2, 3 einen ballonartigen, elastisch komprimierbaren Abschnitt 11, 12, der im gezeigten Ausführungsbeispiel eine im Wesentlichen eiförmige Form im dekomprimierten Zustand aufweist. Ein solcher ballonartiger Abschnitt 11, 12 ist elastisch komprimierbar, wobei zweckmäßigerweise die gesamte Leitungskonfiguration aus PVC gebildet ist, einem Werkstoff, der eine hinreichende Elastizität bietet.

Den beiden ballonartigen Abschnitten 11, 12 ist ein gemeinsames Komprimiermittel 13 zugeordnet, das zwischen den beiden Abschnitten 11, 12 angeordnet ist und zum wechselseitigen Komprimieren der Abschnitte 11, 12 dient. Das Komprimiermittel ist an seiner Außenseite im Wesentlichen der Form eines der Abschnitte 11, 12 entsprechend geformt. Es kann über eine nicht näher gezeigte Mechanik nach oben und unten bewegt werden, wie in den Figuren 1 und 2 ersichtlich. Hierzu wird z.B. ein kleiner Elektromotor oder dergleichen verwendet. Die Steuerung erfolgt über eine Steuerungseinrichtung 14 (siehe Fig. 3), die im gezeigten Ausführungsbeispiel extern zum Gehäuse 10 angeordnet ist.

Ferner sind den jeweiligen Leitungsabschnitten 2, 3 vor und hinter den ballonartigen Abschnitten 11, 12 Mittel 15, 16 zum bedarfsabhängigen Öffnen und Verschließen des jeweiligen Leitungsabschnitts 2, 3 vorgesehen. Diese Mittel 15, die ebenfalls über eine geeignete Mechanik und von der gemeinsamen Steuerungseinrichtung 14 gesteuert betätigt werden und synchron zum Komprimiermittel 13 arbeiten, sind an ihren freien, von außen am jeweiligen Leitungsabschnitt angreifenden Ende im Wesentlichen keilförmig ausgebildet. Jedes Mittel 15, 16 kann aus zwei separat steuerbaren keilförmigen Teilen bestehen, von denen je einer einem Leitungsabschnitt 2, 3 zugeordnet ist. Beide Mittel können aber auch aus einem einstückigen Teil bestehen, das an den Enden keilförmig ist.

Zum Pumpen werden nun mittels des Komprimiermittels 13, das entsprechend der gestrichelten Pfeile A und D bewegbar ist, die beiden Abschnitte 11, 12 wechselseitig zusammengedrückt. Fig. 1 zeigt dabei die Ausgangssituation, in der gerade der Abschnitt 11 zusammengedrückt wurde und nun nachfolgend der Abschnitt 12 komprimiert werden soll. Zu diesem Zweck ist zum einen das dem Abschnitt 12 vorgeschaltete Mittel 15 geschlossen, d.h. der Leitungsabschnitt 3 vor dem ballonartigen Abschnitt 12 ist weitgehend zu, während das nachgeschaltete Mittel 16 offen ist und den Leitungsabschnitt 3 nach dem Abschnitt 12 freigibt. Im Gegenzug ist, siehe Fig. 1, der dem im Moment noch komprimierten ballonartigen Abschnitt 11 nachgeschaltete Leitungsabschnitt 2 über das Mittel 16 geschlossen, während der vorgeschaltete Leitungsabschnitt 2 über das Mittel 15 geöffnet ist. Wird nun das Komprimiermittel 13 bzw. das Druckstück nach oben in Richtung des Pfeils A in Fig. 1 gedrückt, so wird mit zunehmender Bewegung der ballonartige Abschnitt 12 immer weiter komprimiert und die sich in ihm befindliche Flüssigkeit, z.B. Blut aus ihm heraus in die Abfuhrleitung 5 gedrückt, wie durch den gestichelten Flüssigkeitsweg B dargestellt ist. Die Flüssigkeit kann nur in diese Richtung, da ein Rückfluss durch das Verschließen des Leitungsabschnitts 3 über das Mittel 15 ausgeschlossen ist. Auch kann ein Umpumpen in den zunehmend dekomprimierten Abschnitt 11 nicht erfolgen, da der Leitungsabschnitt 2 über das Mittel 16 geschlossen ist. Der ballonartige Abschnitt 11 wird wie beschrieben dekomprimiert, d.h. er entspannt sich und saugt, wie durch den gestrichelten Pfeil C dargestellt ist, über die offene Flüssigkeitszufuhrleitung 4 neue Flüssigkeit nach, d.h. er füllt sich wieder.

Ist nun der ballonartige Abschnitt 12 maximal zulässig komprimiert, d.h. befindet sich das Komprimiermittel 13 in seiner Endstellung, wie sie in Fig. 2 gezeigt ist, so werden die Stellungen der Mittel 15, 16 wie in Fig. 2 gezeigt geändert. Das Mittel 15 schließt die Zufuhrleitung 2 und öffnet die Zufuhrleitung 3, das nachgeschaltete Mittel 16 öffnet die Zufuhrleitung 2 und schließt die Zufuhrleitung 3. Das Komprimiermittel 13 wird nun, siehe Pfeil D in Fig. 2, nach unten bewegt, wodurch der nun gefüllte ballonartige Abschnitt 11 komprimiert und die in ihm befindliche Flüssigkeit aus ihm heraus in Richtung des gestrichelten Pfeils E gefördert wird. Gleichzeitig vergrößert und entspannt sich der bis dato komprimierte Abschnitt 12 wiederum und saugt Flüssigkeit über die Zufuhrleitung 4 nach, siehe den gestrichelten Pfeil F. Die Mittel 15, 16 können den jeweiligen Abschnitt vollständig schließen. Jedoch ist auch ein weitgehendes, jedoch nicht gänzliches Zudrücken ausreichend.

Durch wechselweises Komprimieren und Dekomprimieren der Abschnitte 11, 12 und entsprechendes Schalten der Verschlussmittel 15, 16 kann also ein kontinuierlicher Pumpbetrieb erfolgen. Dabei ist, siehe die Figuren 1 und 2, unter anderem auch durch die gewählte Form des Komprimiermittels 13 bzw. des dieses bildenden Druckstücks, sowie durch eine entsprechende Steuerung des Bewegungswegs desselben sichergestellt, dass die Abschnitte 11, 12 nicht zu weit komprimiert werden. Vielmehr verbleibt in der jeweiligen Endstellung, die in den Figuren 1 und 2 gezeigt ist, stets noch ein hinreichend großes Restvolumen, die einander gegenüberliegende Abschnittswände werden ersichtlich nicht direkt aneinandergedrückt. Hierdurch wird vermieden, dass sensible Flüssigkeitsbestandteile wie z.B. Blutkörperchen oder dergleichen beim Zusammendrücken beschädigt werden. Im Übrigen kann mittels der erfindungsgemäßen Pumpeneinrichtung 1 bedingt durch das beachtlich große Volumen eines Abschnitts 11, 12 eine beachtlich große Flüssigkeitsmenge pro Zeiteinheit gepumpt werden, wobei die Menge natürlich auch abhängig von der eingestellten Frequenz und dem Hub des Komprimiermittels 13 ist.

Fig. 3 zeigt in Form einer Prinzipskizze eine erfindungsgemäße medizinische Behandlungseinrichtung 17 in Form eines Dialysegeräts. Das Dialysegerät umfasst das Gehäuse 10 mit der Pumpeneinrichtung nebst zugeordneter Steuerungseinrichtung 14, die in einem gemeinsamen größeren Pumpeneinrichtungsgehäuse 18 angeordnet sind. In dem Pumpeneinrichtungsgehäuse 18 sind ferner verschiedene Bedienelemente 19 in Form von Knöpfen oder Drehreglern vorgesehen, über die z.B. die entsprechende Betriebsart der medizinischen Behandlungseinrichtung gewählt werden kann, z.B. "Hämofiltration". Ferner kann hierüber das Körpergewicht des Patienten eingegeben werden, über welches dann die Steuerungseinrichtung 14 die erforderliche Blutflussrate errechnet und den Betrieb des Komprimiermittels 13 sowie der Verschlussmittel 15, 16 entsprechend steuert. An einem Display 20 können die entsprechenden Angaben abgelesen oder entsprechende Informationen entnommen werden.

Über ein Anschlussmittel 21 ist die Behandlungseinrichtung 17 mit dem Patienten verbunden, über die Zufuhrleitung 22 wird das vom Patienten kommende Blut dem Pumpengehäuse 18 zugeführt. Über eine entsprechende Rückfuhrleitung 23 wird das gepumpte und filtrierte Blut wiederum dem Patienten zugeführt. Beim Betrieb der Pumpeneinrichtung 1 entsteht am Eingang 21 ein Unterdruck, der vom Widerstand des zum Patienten führenden, dort angeschlossenen Gefäßkatheters sowie der Flussrate der Pumpeneinrichtung 1 abhängig ist. Um den Unterdruck im Leitungssystem auf einen maximal zugelassenen Unterdruck begrenzen zu können, ist eine Einrichtung 24 zum Einstellen und Begrenzen des Unterdrucks vorgesehen. Diese Einrichtung umfasst ein flaschenartiges Behältnis 25, in dem im Falle eines Dialysegeräts eine heparinhaltige Flüssigkeit enthalten ist. Dieses Behältnis 25 hängt ca. 50 - 60 cm unterhalb der Pumpeneinrichtung, wobei über die Höhendifferenz der maximal zulässige Unterdruck definiert und bestimmt wird. Überschreitet der durch den Pumpbetrieb resultierende Unterdruck diesen Maximalwert, so wird automatisch diese heparinhaltige Lösung aus dem Behältnis 25 angesaugt und der Unterdruck ausgeglichen. Über ein Sensorelement 26 wird erfasst, ob noch ein ausreichender Füllstand in dem Behältnis 25 gegeben ist. Falls nicht wird ein Alarmsignal zum Austausch desselben generiert. Weiterhin ist ein Heparinperfusor 27 vorgesehen, über den kontinuierlich Heparin über einen entsprechenden Anschluss in die Zufuhrleitung 22 gegeben wird, um eine Verstopfung des Schlauchsystems mit Blut zu vermeiden. Eine solche Verstopfung kann bei dem erfindungsgemäßen Gerät infolge der Verwendung der erfindungsgemäßen Pumpeneinrichtung jedoch weitgehend ausgeschlossen werden, da die Fließgeschwindigkeit in den Leitungen, in denen Blut transportiert wird, relativ hoch ist, weil infolge der Verwendung der beachtlich großdimensionierten ballonartigen Abschnitte eine relativ hohe Fließgeschwindigkeit erreicht wird. Zudem sollten möglichst dünne Leitungen innerhalb des gesamten Leitungssystems verwendet werden, was einer hohen Fließgeschwindigkeit ebenfalls zuträglich ist.

Das aufgrund des Pumpbetriebs vom Patienten geförderte Blut wird über die Zufuhrleitung 22 in eine Luftfalle 28 gefördert, wo etwaige im Blut befindliche Luft abgeschieden und über ein entsprechendes Ablassventil 29 im Bedarfsfall abgezogen werden kann. Die Zufuhrleitung 22 mündet ersichtlich von unten in die Luftfalle 28 und verlässt diese von oben nach unten laufend, es ergibt sich also im Wesentlichen die Form eines "umgekehrten Y". Da Luft bekanntermaßen nach oben steigt wird hierdurch vermieden, dass die Luft in irgendeiner Form erneut in das Leitungssystem gelangen kann. Über ein der Luftfalle nachgeschaltetes Sensorelement 30, z.B. einen fotometrischen Sensor wird überprüft, ob im Blut noch Luft vorhanden ist. Falls ja wird sofort ein Alarmsignal gegeben und der Betrieb der Pumpeneinrichtung gestoppt. Das Sensorelement 30 ist extern zur Leitung 22 angeordnet und erfasst den Luftgehalt durch die Leitung hindurch.

Anschließend mündet die Zufuhrleitung 22 in der Pumpeneinrichtung 1 bzw. an dem entsprechenden Anschlussmittel 6. Die abgehende Abfuhrleitung 31, die am Anschlussmittel 7 angeschlossen ist, mündet anschließend in einem Hämofiltrationsfilter 32, der an einer drehbaren Filterhaltung 33 angeordnet ist. Im Dialysebetrieb wird der Hämofiltrationsfilter 32 in eine im Wesentlichen horizontale Position gedreht, zur Vorbereitung der Einrichtung und zur Entlüftung des Filters wird er in eine senkrechte Position gebracht. Die horizontale Position ist für einen gleichmäßigen Pumpbetrieb, eine gleichmäßige Belastung des Filters und einen niedrigen Pumpendruck zweckmäßig.

Im Hämofiltrationsfilter 32 abgefilterter Urin gelangt über eine Leitung 34 in einen Urinsammelbeutel 35. Ferner ist ein Behältnis 36 mit einer Substitutionslösung vorgesehen, die über eine Pumpe 37 dem filtrierten Blut zugeführt werden kann und die abgeschiedene Flüssigkeitsmenge, die der gesammelte Urin bildet, ersetzt. Schließlich ist ein weiteres Behältnis 38 mit einer Dialisatlösung vorgesehen, die über eine Pumpe 39 in Gegenrichtung des Blutflusses zugeführt werden kann.

Nach dem Vorfüllen und Entlüften der Leitungen des Dialysegeräts gemäß Fig. 1 und insbesondere des Hämofiltrationsfilters 32 mit heparinhaltiger Lösung, was durch Betätigen einer der Bedientasten 19 erfolgt, wird die Einrichtung über die Anschlüsse 21, 23 mit großlumigen Kathetern verbunden, welche in entsprechenden Gefäßen des Patienten angebracht sind. Während des Vorfüll- oder Lüftungsprozesses ist das Sensorelement 30 deaktiviert, es werden keine Alarme ausgelöst. Nach Anbringen der Unterdruckkompensationseinrichtung 24, die zweckmäßigerweise mit 0,9 % NaCI und Heparin gefüllt ist, wird über eines der Betätigungselemente 19 die gewünschte Betriebsart, also z.B. "Hämofiltration" gewählt und das Körpergewicht des Patienten eingegeben. Möchte man eine geringere oder größere Blutflussrate haben, so wird ein entsprechend geringeres oder größeres Körpergewicht eingestellt.

Anfallende Luft kann wie bereits beschrieben aus der Luftfalle 28 abgezogen werden. Wird vom Sensorelement 30 jedoch Luft festgestellt, so wird der Pumpenbetrieb sofort gestoppt und ein Daueralarm ausgelöst. Das Leitungssystem muss dann bis zum Anschluss an den Hämofiltrationsfilter 32 erneut entlüftet werden, die Entlüftung kann aber auch über einen entsprechenden Anschluss 40 erfolgen.

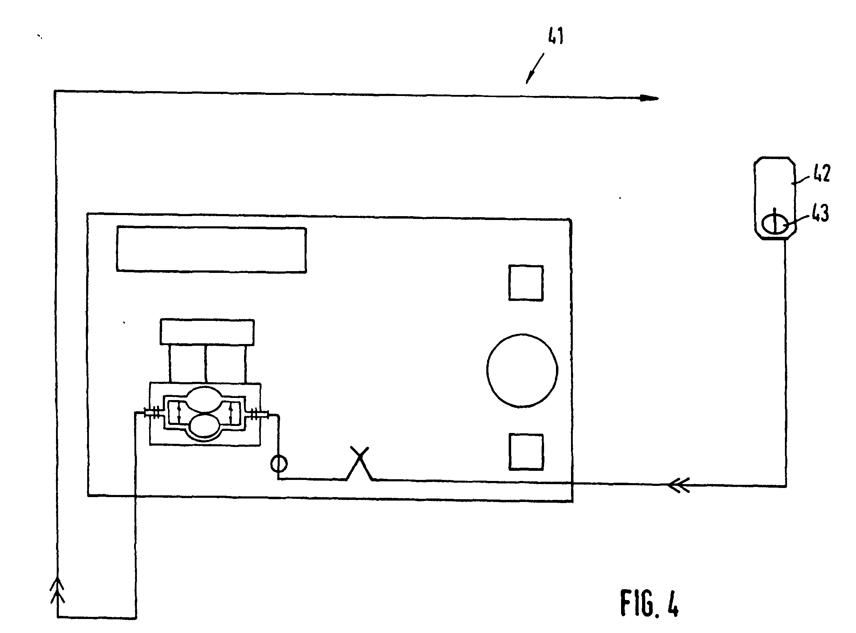

Fig. 4 zeigt schließlich eine weitere erfindungsgemäße medizinische Behandlungseinrichtung 41 in Form einer Infusionsapparatur. Eine Flasche 42 mit einer zuzuführenden Flüssigkeit, z.B. einer Ernährungslösung zur enteralen oder parenteralen Ernährung oder für die Zufuhr in den Kreislauf, in die Bauchhöhle oder in Gelenke wird in einer Position oberhalb der Pumpeneinrichtung aufgehängt. Auch hier ist ein Füllstandssensorelement 43 vorgesehen, das einen entsprechenden Austausch der Flasche signalisiert. Nach Vorfüllen des Systems wird über ein geeignetes Bedienelement die Betriebsart "kontinuierliche oder intermetierende Ernährung" gewählt, und schließlich die Flussrate oder die Bolusmenge eingestellt.

Im Falle einer Betriebsart "Bauchlavage" wird statt einer Flasche 42 ein mehrere Liter enthaltender Beutel mit einer Lösung verwendet. Über eines der Bedienelemente wird die entsprechende Betriebsart "Bauchlavage" eingestellt und die Flussrate eingegeben.

Im Falle einer Arthroskopie beispielsweise zur Spülung des Kniegelenks, der Bauchhöhle oder anderer Höhlen während der Operation wird ein System wie bei einer Bauchlavage verwendet. Jedoch wird hier der Start und der Stopp der Pumpeneinrichtung über ein entsprechendes beispielsweise mit dem Fuß zu betätigendes Pedal, das kabellos oder über ein Kabel mit der Steuerungseinrichtung der Pumpeneinrichtung gekoppelt ist, gesteuert.

## Patentansprüche

1. Pumpeneinrichtung zum Pumpen einer Flüssigkeit, insbesondere einer einem menschlichen oder tierischen Körper zuzuführenden Flüssigkeit, insbesondere von Blut, umfassend wenigstens einen die Flüssigkeit führenden schlauchartigen Leitungsabschnitt (2, 3) mit einem ballonartigen, elastisch komprimierbaren Abschnitt (11, 12) sowie ein Mittel (13) zum zeitweisen Komprimieren des ballonartigen Abschnitts (11, 12) zur Verringerung seines Volumens, **dadurch gekennzeichnet, dass** das Komprimiermittel (13) ein am ballonartigen Abschnitt (11, 12) außenseitig angreifendes, gesteuert motorisch bewegbares Druckstück aufweist, und die Außenform des Druckstücks im am ballonartigen Abschnitt (11, 12) angreifenden Bereich im Wesentlichen der expandierten Form des gegenüberliegenden Wandabschnitts des ballonartigen Abschnitts (11, 12) entspricht, wobei der ballonartige Abschnitt (11, 12) über das Komprimiermittel (13) derart komprimierbar ist, dass die einander gegenüberliegenden Abschnittswände nicht aneinander gedrückt werden.

2. Pumpeneinrichtung nach Anspruch 1, **dadurch gekennzeichnet, dass** zwei schlauchartige Leitungsabschnitte (2, 3) mit je einem ballonartigen, elastisch komprimierbaren Abschnitt (11, 12) vorgesehen sind, die von einer gemeinsamen Zufuhrleitung (4) abgehen und die in eine gemeinsame Ableitung (5) münden.

3. Pumpeneinrichtung nach Anspruch 2, **dadurch gekennzeichnet, dass** den beiden elastisch komprimierbaren Abschnitte (11, 12) je ein separates Komprimiermittel zugeordnet ist, oder dass beide über ein gemeinsames Komprimiermittel (13) betätigbar sind.

4. Pumpeneinrichtung nach Anspruch 2 oder 3, **dadurch gekennzeichnet, dass** die beiden Abschnitte (11, 12) über das oder die Komprimiermittel (13) gleichzeitig oder wechselweise komprimierbar sind.

5. Pumpeneinrichtung nach einem der vorangehenden Ansprüche, **dadurch gekennzeichnet, dass** zwei schlauchartige Leitungsabschnitte (2, 3) mit je einem ballonartigen elastisch komprimierbaren Abschnitt (11, 12) vorgesehen sind, die einander gegenüberliegend angeordnet sind und zwischen denen ein oder zwei bewegbare Druckstücke angeordnet sind.

6. Pumpeneinrichtung nach einem der vorangehenden Ansprüche, **dadurch gekennzeichnet, dass** eine Steuerungseinrichtung (14) zum Steuern des Betriebs des oder der Komprimiermittels (13) vorgesehen ist.

7. Pumpeneinrichtung nach Anspruch 6, **dadurch gekennzeichnet, dass** über die Steuerungseinrichtung (14) die Frequenz und/oder die Amplitude der Komprimierbewegungen des oder der Komprimiermittel (13) variierbar ist.

8. Pumpeneinrichtung nach Anspruch 6 oder 7, **dadurch gekennzeichnet, dass** an der Steuerungseinrichtung (14) Mittel zum Wählen und Einstellen der Betriebsparameter des oder der Komprimiermittel (13) und/oder von für den Betrieb des oder der Komprimiermittel (13) relevanten Parametern, insbesondere des Körpergewichts des Menschen oder des Tiers vorgesehen oder zugeordnet sind.

9. Pumpeneinrichtung nach einem der vorangehenden Ansprüche, **dadurch gekennzeichnet, dass** ein Leitungsabschnitt (2, 3) samt dem ballonartigen Abschnitt (11, 12) aus PVC ist.

10. Pumpeneinrichtung nach einem der vorangehenden Ansprüche, **dadurch gekennzeichnet, dass** vor und/oder hinter dem ballonartigen Abschnitt (11, 12) Mittel (15, 16) zum reversiblen, zumindest teilweisen Verschließen und Öffnen des Leitungsabschnitts (2, 3) vorgesehen sind.

11. Pumpeneinrichtung nach Anspruch 10, **dadurch gekennzeichnet, dass** bei zwei Leitungsabschnitten (2, 3) mit je einem ballonartigen Abschnitt (11, 12) jedem Leitungsabschnitt (2, 3) vor und hinter dem jeweiligen ballonartigen Abschnitt (11, 12) ein solches Mittel (15, 16) zugeordnet ist.

12. Pumpeneinrichtung nach Anspruch 11, **dadurch gekennzeichnet, dass** bei einer wechselweisen Komprimierung der beiden Abschnitte (11, 12) das dem zu komprimierenden Abschnitt vorgeschaltete Mittel (15) und das dem nicht zu komprimierenden Abschnitt nachgeschaltete Mittel (16) zumindest teilweise geschlossen und die jeweils anderen Mittel (15, 16) geöffnet werden.

13. Pumpeneinrichtung nach Anspruch 11, **dadurch gekennzeichnet, dass** bei einer gleichzeitigen Komprimierung der beiden Abschnitte die den ihnen vorgeschalteten Mittel geschlossen und die nachgeschalteten Mittel geöffnet werden.

14. Pumpeneinrichtung nach einem der Ansprüche 10 bis 13, **dadurch gekennzeichnet, dass** ein Mittel (15, 16) ein von Außen auf den Leitungsabschnitt (2, 3) drückendes bewegbares Druckelement umfasst.

15. Pumpeneinrichtung nach Anspruch 14, **dadurch gekennzeichnet, dass** ein Druckelement eine im Wesentlichen keilförmige Form im Bereich seines am Leitungsabschnitt (2, 3) angreifenden Endes aufweist.

16. Pumpeneinrichtung nach einem der Ansprüche 10 bis 15, **dadurch gekennzeichnet, dass** der Betrieb der Mittel (15, 16) über die Steuerungseinrichtung (14) steuerbar ist.

17. Pumpeneinrichtung nach einem der vorangehenden Ansprüche, **dadurch gekennzeichnet, dass** der oder die Leitungsabschnitte (2, 3) sowie das oder die Komprimiermittel (13) in einem separaten Gehäuse (10) untergebracht sind, an dem Anschlüsse (6, 7) für von außerhalb des Gehäuses (10) zugeführte Zufuhr- und Abfuhrleitungen (22, 31) für die zu pumpende Flüssigkeit vorgesehen sind.

18. Pumpeneinrichtung nach Anspruch 17, **dadurch gekennzeichnet, dass** der oder die Leitungsabschnitte (2, 3) lösbar in dem Gehäuse (10) angeordnet sind.

19. Pumpeneinrichtung nach Anspruch 17 oder 18, **dadurch gekennzeichnet, dass** ein Einrichtungsgehäuse (18) vorgesehen ist, in dem das Gehäuse (10) lösbar angeordnet und mit dort vorgesehenen Zufuhr- und Abfuhrleitungen (22, 31) verbindbar ist, und in dem die Steuerungseinrichtung (14) angeordnet ist, die an am Gehäuse (10) vorgesehene Steuerungsanschlüsse anschließbar ist.

20. Medizinische Behandlungseinrichtung zum Zuführen einer Flüssigkeit zum menschlichen oder tierischen Körper, insbesondere Dialyse- oder Infusionseinrichtung, umfassend eine Pumpeneinrichtung nach einem der Ansprüche 1 bis 19.

21. Behandlungseinrichtung nach Anspruch 20, **dadurch gekennzeichnet, dass** in der Zufuhrleitung (22) der Flüssigkeit zu der Pumpeneinrichtung (1) eine Einrichtung (24) zum Einstellen und Begrenzen eines durch den Betrieb der Pumpeneinrichtung (1) am Einlass (21) der Zufuhrleitung (22) entstehenden Unterdrucks vorgesehen oder in diese einschaltbar ist.

22. Behandlungseinrichtung nach Anspruch 21, **dadurch gekennzeichnet, dass** die Einrichtung (24) ein mit einer biokompatiblen Flüssigkeit gefülltes Behältnis (25) umfasst, das über eine Leitung mit der Zufuhrleitung (22) in Verbindung steht und das an einer Position unterhalb der Pumpeneinrichtung (1) angeordnet oder anbringbar ist.

23. Behandlungseinrichtung nach Anspruch 22, **dadurch gekennzeichnet, dass** ein Sensormittel (26) zum Erfassen des Füllstands des Behältnisses (25) vorgesehen ist, das ein Erfassungssignal liefert, in dessen Abhängigkeit ein Alarmsignal über die Steuerungseinrichtung (14) gebbar und/oder der Betrieb der Pumpeneinrichtung (1) steuerbar ist.

24. Behandlungseinrichtung nach einem der Ansprüche 20 bis 23, **dadurch gekennzeichnet, dass** in der Zufuhrleitung (22) zur Pumpeneinrichtung (1) eine Luftfalle (28) und dieser nachgeschaltet ein Sensorelement (30) zum Ermitteln von Luft in der Flüssigkeit angeordnet ist.

25. Behandlungseinrichtung nach Anspruch 24, **dadurch gekennzeichnet, dass** die der Luftfalle (28) zugeführte Flüssigkeit über einen von unten her in die Luftfalle (28) führenden Zufuhrleitungsabschnitt zugeführt und über einen nach unten von der Luftfalle (28) abgehenden Abfuhrleitungsabschnitt abgeführt wird.

## Claims

1. Pumping device for pumping a fluid, in particular a fluid to be supplied to a human or animal body, in particular blood, comprising at least one hose-like line section (2, 3) guiding the fluid and having a balloon-like, resiliently compressible section (11, 12) and means (13) for periodic compression of the balloon-like section (11, 12) in order to reduce its volume, **characterised in that** the compression means (13) has a pressure member movable motorically in a controlled manner and acting on the exterior of the balloon-like section (11, 12), and the external form of the pressure member in region acting on the balloon-like section (11, 12) substantially corresponds to the expanded shape of the opposing wall section of the balloon-like section (11, 12), the balloon-like section (11, 12) being compressible via the compression means (13) in such a manner that the opposing section walls are not pressed against one another.

2. Pumping device according to claim 1, **characterised in that** two hose-like line sections (2, 3) are each provided with a balloon-like, resiliently compressible section (11, 12) which branch off from a common supply line (4) and open into a common discharge line (5).

3. Pumping device according to claim 2, **characterised in that** a respectively separate compression means is allocated to each of the two resiliently compressible sections (11, 12), or both are actuatable via a common compression means (13).

4. Pumping device according to claim 2 or 3, **characterised in that** the two sections (11, 12) are compressible simultaneously or alternately by the one or more compression means (13).

5. Pumping device according to one of the preceding claims, **characterised in that** two hose-like line sections (2, 3) are provided with a respective balloon-like, resiliently compressible section (11, 12), which are disposed opposite one another and are disposed between the one or two movable pressure members.

6. Pumping device according to one of the preceding claims, **characterised in that** a control device (14) is provided for controlling the operation of the one or more compression means (13).

7. Pumping device according to claim 6, **characterised in that** the frequency and/or the amplitude of the compression movements of the one or more compression means (13) is variable via the control device (14).

8. Pumping device according to claim 6 or 7, **characterised in that** one the control device (14) means are provided or allocated for selecting or setting the operating parameters of the one or more compression means (13) and/or of the relevant parameters for the operation of the one or more compression means (13), in particular the body weight of the person or animal.

9. Pumping device according to one of the preceding claims, **characterised in that** a line section (2, 3) together with the balloon-like section (11, 12) is composed of PVC.

10. Pumping device according to one of the preceding claims, **characterised in that** upstream and/or downstream of the balloon-like section (11, 12) means (15, 16) are provided for the reversible, at least partial closing and opening of the line section (2, 3).

11. Pumping device according to claim 10, **characterised in that** in the case of two line sections (2, 3) each having a balloon-like section (11, 12), such a means (15, 16) is allocated to each line section (2, 3) upstream and downstream of the respective balloon-like section (11, 12).

12. Pumping device according to claim 11, **characterised in that** in the case of alternate compression of the two sections (11, 12) the means (15) connected upstream of the section to be compressed and the means (16) connected downstream of the section not to be compressed are at least partly closed and the respective other means (15, 16) are open.

13. Pumping device according to claim 11, **characterised in that** in the case of simultaneous compression of the two sections, the means connected upstream thereof are closed and the means connected downstream are open.

14. Pumping device according to one of claims 10 to 13, **characterised in that** a means (15, 16) comprises a movable pressure element which can be pressed on to the line section (2, 3) from outside.

15. Pumping device according to claim 14, **characterised in that** a pressure element has a substantially wedge-like shape in the region of its end acting on the line section (2, 3).

16. Pumping device according to one of claims 10 to 15, **characterised in that** the operation of the means (15, 16) is controllable via the control device (14).

17. Pumping device according to one of the preceding claims, **characterised in that** the one or more line sections (2, 3) and the one or more compression means (13) are housed in a separate housing (10), on which terminals (6, 7) are provided for supply and discharge lines (22, 31) supplied from outside the housing for the fluid to be pumped.

18. Pumping device according to claim 17, **characterised in that** the one or more line sections (2, 3) are disposed detachably in the housing (10).

19. Pumping device according to claim 17 or 18, **characterised in that** a device housing (18) is provided, in which the housing (10) is detachably disposed and is connectable to supply and discharge lines (22, 31) provided there, and in which the control device (14) is disposed, which is connectable to the control terminals provided on the housing (10).

20. Medical treatment device for supplying a fluid to the human or animal body, in particular dialysis or infusion device, comprising a pumping device according to one of claims 1 to 19.

21. Treatment device according to claim 20, **characterised in that** in the supply line (22) of the fluid to the pumping device (1) a device (24) is provided or may be connected for setting and limiting a low pressure arising from the operation of the pumping device (1) at the inlet (21) of the supply line (22).

22. Treatment device according to claim 21, **characterised in that** the device (24) comprises a vessel (25) filled with a biocompatible fluid, which communicates via a line with the supply line (22) and which is disposed in or movable to a position below the pumping device (1).

23. Treatment device according to claim 22, **characterised in that** a sensor means (26) is provided for detecting the fill level of the vessel (25), which delivers a detection signal according to which an alarm signal may be transmitted via the control device (14) and/or the operation of the pumping device (1) may be controlled.

24. Treatment device according to one of claims 20 to 23, **characterised in that** in the supply line (22) for the pumping device (1) an air trap (28) is disposed and downstream thereof a sensor element (30) is disposed for detecting air in the fluid.

25. Treatment device according to claim 24, **characterised in that** the fluid supplied to the air trap (28) is supplied via a supply line section leading from below into the air trap (28) and is discharged via a discharge line section branching off down from the air trap (28).

## Revendications

1. Système de pompe destiné à pomper un liquide, en particulier un liquide à administrer dans un corps humain ou animal, en particulier du sang, comportant au moins une partie de conduite (2, 3) de type tuyau flexible amenant le liquide et munie d'une partie (11, 12) de type ballonnet élastiquement compressible, ainsi qu'un moyen (13) pour comprimer périodiquement la partie (11, 12) de type ballonnet en vue de réduire son volume, **caractérisé en ce que** le moyen de compression (13) comporte une pièce de pression, entrant en contact du côté extérieur sur la partie (11, 12) de type ballonnet et apte à être déplacée de manière commandée par un moteur, et la forme extérieure de la pièce de pression dans la zone entrant en contact avec la partie (11, 12) de type ballonnet correspond sensiblement à la forme expansée de la partie de paroi opposée de la partie (11, 12) de type ballonnet, ladite partie (11, 12) de type ballonnet pouvant être comprimée par l'intermédiaire du moyen de compression (13), de telle sorte que les parties de paroi face à face sont poussées l'une contre l'autre.

2. Système de pompe selon la revendication 1, **caractérisé en ce qu'**il est prévu deux parties de conduite (2, 3) de type tuyau flexible, comportant chacune une partie (11, 12) de type ballonnet élastiquement compressible, lesquelles partent d'une conduite d'admission (4) commune et débouchent dans une conduite d'évacuation (5) commune.

3. Système de pompe selon la revendication 2, **caractérisé en ce qu'**un moyen de compression séparé est affecté à chacune des deux parties (11, 12) élastiquement compressibles, ou **en ce que** lesdites deux parties peuvent être actionnées par l'intermédiaire d'un moyen de compression (13) commun.

4. Système de pompe selon la revendication 2 ou 3, **caractérisé en ce que** les deux parties (11, 12) peuvent être comprimées simultanément ou en alternance par l'intermédiaire du ou des moyen(s) de compression (13).

5. Système de pompe selon l'une quelconque des revendications précédentes, **caractérisé en ce qu'**il est prévu deux parties de conduite (2, 3) de type tuyau flexible, munies chacune d'une partie (11, 12) de type ballonnet élastiquement compressible, lesquelles sont disposées en regard l'une de l'autre et entre lesquelles sont disposées une ou deux pièces de pression mobiles.

6. Système de pompe selon l'une quelconque des revendications précédentes, **caractérisé en ce qu'**il est prévu un dispositif de commande (14) pour commander le fonctionnement du ou des moyen(s) de compression (13).

7. Système de pompe selon la revendication 6, **caractérisé en ce que**, par l'intermédiaire du dispositif de commande (14), il est possible de faire varier la fréquence et/ou l'amplitude des mouvements de compression du ou des moyen(s) de compression (13).

8. Système de pompe selon la revendication 6 ou 7, **caractérisé en ce que** des moyens pour sélectionner et régler les paramètres de service du ou des moyen(s) de compression (13) et/ou de paramètres significatifs du fonctionnement du ou des moyen(s) de compression (13), en particulier le poids du corps humain ou animal, sont prévus sur le dispositif de commande (14) ou sont associés à celui-ci.

9. Système de pompe selon l'une quelconque des revendications précédentes, **caractérisé en ce qu'**une partie de conduite (2, 3), y compris la partie (11, 12) de type ballonnet, est réalisée en PVC.

10. Système de pompe selon l'une quelconque des revendications précédentes, **caractérisé en ce que** des moyens (15, 16) pour la fermeture et l'ouverture réversibles, du moins partiellement, de la partie de conduite (2, 3) sont prévus en amont et/ou en aval de la partie (11, 12) de type ballonnet.

11. Système de pompe selon la revendication 10, **caractérisé en ce que**, en présence de deux parties de conduite (2, 3) comportant chacune une partie (11, 12) de type ballonnet, un tel moyen (15, 16) est affecté à chaque partie de conduite (2, 3) en amont et en aval de la partie (11, 12) de type ballonnet respective.

12. Système de pompe selon la revendication 11, **caractérisé en ce que**, en cas de compression alternée des deux parties (11, 12), le moyen (15), monté en amont de la partie à comprimer, et le moyen (16), monté en aval de la partie à comprimer, sont au moins partiellement fermés et les respectivement autres moyens (15, 16) sont ouverts.

13. Système de pompe selon la revendication 11, **caractérisé en ce que**, en cas de compression simultanée des deux parties, les moyens qui sont montés en amont des deux parties sont fermés et les moyens montés en aval sont ouverts.

14. Système de pompe selon l'une quelconque des revendications 10 à 13, **caractérisé en ce qu'**un moyen (15, 16) comporte un élément de pression mobile, qui presse de l'extérieur sur la partie de conduite (2, 3).

15. Système de pompe selon la revendication 14, **caractérisé en ce qu'**un élément de pression a une forme sensiblement conique dans la zone de son extrémité entrant en contact avec la partie de conduite (2,3).

16. Système de pompe selon l'une quelconque des revendications 10 à 15, **caractérisé en ce que** le fonctionnement des moyens (15, 16) peut être commandé par l'intermédiaire du dispositif de commande (14).

17. Système de pompe selon l'une quelconque des revendications précédentes, **caractérisé en ce que** la ou les partie(s) de conduite (2,3), ainsi que le ou les moyen(s) de compression (13) sont logés dans un boîtier (10) séparé, sur lequel sont prévus des raccords (6, 7) pour des conduites d'admission et d'évacuation (22, 31) du liquide à pomper, guidées à l'extérieur du boîtier (10).

18. Système de pompe selon la revendication 17, **caractérisé en ce que** le ou les partie(s) de conduite (2,3) sont montées amovibles dans le boîtier (10).

19. Système de pompe selon la revendication 17 ou 18, **caractérisé en ce qu'**il est prévu un boîtier du système (18), dans lequel est monté amovible le boîtier (10) et lequel est apte à être relié à des conduites d'admission et d'évacuation (22, 31) qui sont prévues sur ce dernier, et dans lequel est monté le dispositif de commande (14), qui peut être raccordé à des raccords de commande prévus sur le boîtier (10).

20. Dispositif de traitement médical destiné à administrer un liquide dans le corps humain ou animal, en particulier dispositif de dialyse ou de perfusion, comportant un système de pompe selon l'une quelconque des revendications 1 à 19.

21. Dispositif de traitement selon la revendication 20, **caractérisé en ce qu'**un dispositif (24), destiné à régler et limiter une dépression générée par le fonctionnement du système de pompe (1) au niveau de l'entrée (21) de la conduite d'admission (22), est prévu ou peut être monté dans la conduite d'admission (22), acheminant le liquide vers le système de pompe (1).

22. Dispositif de traitement selon la revendication 21, **caractérisé en ce que** le dispositif (24) comporte un récipient (25), qui contient un liquide biocompatible et qui communique via une conduite avec la conduite d'admission (22) et est disposé ou peut être monté à un emplacement en dessous du système de pompe (1).

23. Dispositif de traitement selon la revendication 22, **caractérisé en ce qu'**il est prévu un moyen de détection (26), qui est destiné à détecter le niveau de remplissage du récipient (25) et qui délivre un signal de détection, en fonction duquel un signal d'alarme peut être émis par l'intermédiaire du dispositif de commande (14) et/ou en fonction duquel le fonctionnement du système de pompe (1) peut être commandé.

24. Dispositif de traitement selon l'une quelconque des revendications 20 à 23, **caractérisé en ce que** dans la conduite d'admission (22) menant vers le système de pompe (1) sont disposés un piège à air (28) et un élément de détection (30), monté en aval de ce dernier et destiné à détecter l'air dans le liquide.

25. Dispositif de traitement selon la revendication 24, **caractérisé en ce que** le liquide acheminé vers le piège à air (28) est acheminé par l'intermédiaire d'un tronçon de la conduite d'admission menant par le bas dans le piège à air (28) et est évacué par l'intermédiaire d'un tronçon de la conduite d'évacuation partant du piège à air (28) vers le bas.
